# EUROPÄISCHE PATENTSCHRIFT

(11) **EP 3 922 162 B1**
(45) Veröffentlichungstag und Bekanntmachung des Hinweises auf die Patenterteilung: **08.03.2023**
(21) Anmeldenummer: 21176690.2
(22) Anmeldetag: 28.05.2021
(51) Int. Cl.: A61B 1/00, A61B 1/06, A61B 1/07, A61B 1/05

(54) **ENDOSKOP MIT SCHWENKBARER BILDERFASSUNGSEINRICHTUNG**
ENDOSCOPE WITH PIVOTABLE IMAGING DEVICE
ENDOSCOPE POURVU DE DISPOSITIF PIVOTANT DE CAPTURE D'IMAGE

(30) Priorität: 09.06.2020 DE 102020115258
(43) Veröffentlichungstag der Anmeldung: 15.12.2021
(73) Patentinhaber: Karl Storz SE & Co. KG, 78532 Tuttlingen (DE)
(72) Erfinder: Forster, Jonas, 78532 Tuttlingen (DE); Heni, Andreas, 78532 Tuttlingen (DE); Kupferschmid, Markus, 78532 Tuttlingen (DE); Ulmschneider, Daniel, 78532 Tuttlingen (DE)

(56) Entgegenhaltungen:
- DE-A1-102010 028 147
- DE-A1-102013 217 449
- DE-A1-102017 100 056
- US-A1- 2013 182 091

## Beschreibung

Die vorliegende Erfindung ist auf ein Endoskop oder Exoskop mit schwenkbarer Bilderfassungseinrichtung bezogen.

Neben Endoskopen mit Blickrichtung parallel zur Längsachse des Schafts ("Geradeausblick") werden vor allem Endoskope, deren Blickrichtung mit der Längsachse des Schafts einen Winkel einschließen, der oft im Bereich von 30° bis 80° liegt, ("Vorausblick") verwendet. Für manche Anwendungen kommen auch Endoskope mit einem einstellbaren Winkel zwischen Blickrichtung und Längsachse des Schafts in Betracht.

In US 2007/0055103 A1 ist ein Endoskop mit variabler Blickrichtung beschrieben (Titel, Absätze [0002], [0017], [0018]). Ein optisches Abbildungssystem ("optical imaging system") 18 mit einer Abbildungsoptik ("imaging optics") 22 und einem CCD-Chip 24 ist in einem starren distalen Kopf ("rigid distal head") 16 des Endoskops 10 angeordnet (Absätze [0073], [0074]). Der distale Kopf 16 ist um eine erste Schwenkachse ("first pivot axis") 38 schwenkbar mit einem ersten Schaftabschnitt ("first shaft portion") 32 verbunden, der erste Schaftabschnitt 32 ist um eine zweite Schwenkachse 40 schwenkbar mit einem zweiten Schaftabschnitt 34 verbunden (Absätze [0079], [0082], [0083]).

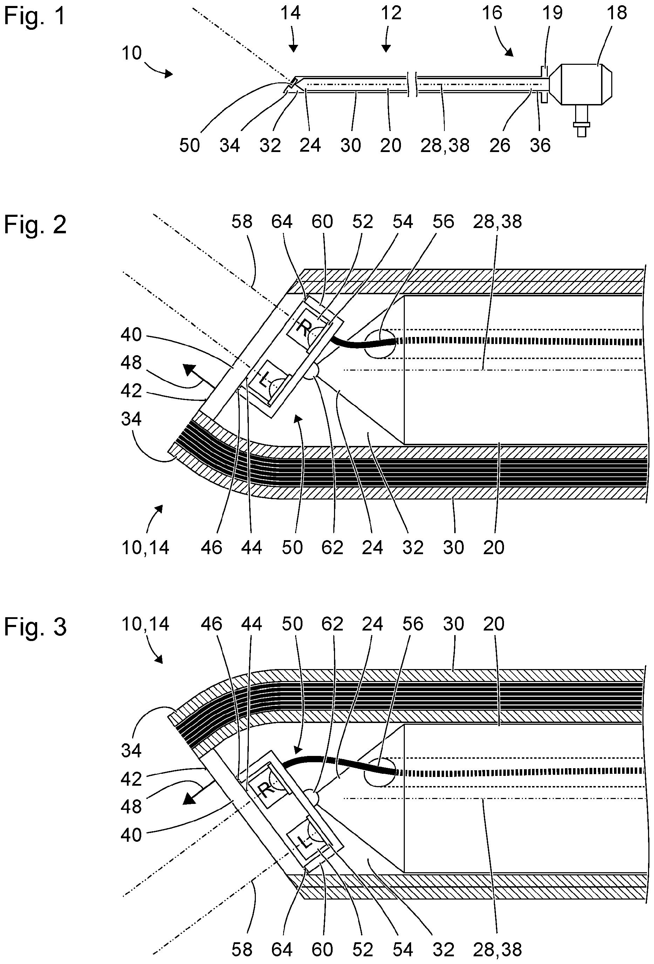

In DE 10 2010 028 147 A1 ist ein Endoskop mit variabler Blickrichtung beschrieben. Im distalen Bereich eines Endoskopschafts 1 ist eine bewegliche Plattform 5 mit einem CCD-Chip 6 und einem Linsensystem 7 vorgesehen. Die Plattform 5 ist über zug- und schubfeste und längsaxial verschiebbare Verschiebeelemente 8, 8', 8" oder Koppelstangen mit einem oder mehreren Antrieben 12 verbunden, die ein Kippen der Plattform 5 ermöglichen (Absätze [0008], [0035] bis [0038], Figur 1).

In DE 10 2012 206 963 A1 ist ein Endoskop beschrieben, das einen drehbar gelagerten Halter 5 mit einer Digitalkamera 4 an oder in einer Halterung 6 aufweist, wobei die Halterung 6 radial drehbar so angeordnet ist, dass die Drehachsen der Halterung 6 und des Halters 5 senkrecht zueinander angeordnet sind (Absatz [0008]).

In US 2013/0182091 A1 ist ein Endoskop mit einer veränderbaren Blickrichtung beschrieben (Zusammenfassung, Absatz [0002]). Eine Abbildungseinheit ("imaging unit") 12 ist angetrieben durch Antriebsstäbe 22, 23 von zwei Antriebskraft-Übertragungsmechanismen um zwei verschiedene Achsen X, Y rotierbar (Absätze [0002], [0028], [0062], Fig. 3, 4, 6, 8, 9).

In US 2014/0249369 A1 ist ein Endoskop mit bewegbarem Blickfeld beschrieben. In einem distalen Ende eines Schafts C des Endoskops ist ein kugelförmiges Gehäuse 1 mit einer bildmäßige Lichtempfangseinrichtung 5 angeordnet (Absätze [0027], [0033], Figuren 2, 3). Das kugelförmige Gehäuse 5 ist mittels Steuerdrähten 3 schwenkbar (Absätze [0029], [0036], Figuren 2, 3).

In US 2015/0359420 A1 ist ein Endoskop 1 mit einem teilweise flexiblen Schaft ("insertion section") 11 und einer Abbildungseinheit ("imaging unit") 36 am distalen Ende ("tip end portion") des Schafts 11 beschrieben (Absatz [0036], Fig. 1). Die Abbildungseinheit 6a ist schwenkbar angeordnet (Absätze [0079], [0081], Fig. 4, 5).

In EP 3 243 426 A1 ist ein Endoskop 100 mit einem länglichen Schaft ("elongated shaft") 101 und einer Bildsensoranordnung ("image sensor assembly") 202 beschrieben (Titel, Zusammenfassung, Absätze [0002], [0008], [0019], [0022], Fig. 1, 2, 5). Die Bildsensoranordnung 202 kann um eine laterale Gelenkachse ("lateral articulation axis") T1 geschwenkt werden (Absatz [0026], Fig. 2, 3, 5, 6, 7).

In DE 10 2017 103 721 A1 ist ein Stereo-Endoskop beschrieben, bei dem jeder der beiden optischen Kanäle ein Objektiv 40, 50 und eine Blickrichtungseinrichtung 20, 30 umfasst. Jede Blickrichtungseinrichtung 20, 30 ist um eine zugehörige ortsfeste Rotationsachse 48, 58 rotierbar. Eine Antriebswelle 90 ist durch Zahnräder mit den Blickrichtungseinrichtungen 20, 30 gekoppelt, um diese zu rotieren. Kurvengetriebe verschieben simultan die Blickrichtungseinrichtungen 20, 30, die Objektive 40, 50 und Bildsensoren 60, 70 parallel zu den Rotationsachsen.

In WO 2018/065241 A1 ist ein Stereo-Videoendoskop 2 mit einem optischen System 20 beschrieben (Titel, Zusammenfassung, Seite 13, Zeile 26, bis Seite 14, Zeile 7, Seite 14, Zeilen 27 bis 29, Fig. 1, 2). Durch Drehen eines Handgriffs 4 kann die Blickrichtung um die Längsachse des Endoskopschafts 6 rotiert werden (Seite 14, Zeilen 18 bis 21). Zur Beibehaltung der Horizontallage des angezeigten Bilds wird bei einer Drehung des Handgriffs 4 ein Drehrad 14 festgehalten, dies bewirkt, dass die Bildsensoren 52L, 52R im Inneren des Endoskopschafts 6 die Drehbewegung nicht mitvollziehen (Seite 14, Zeilen 21 bis 25, Seite 16, Zeilen 2 bis 13, Fig. 2).

In US 2019/0274526 A1 ist ein Stereoendoskop 1 mit einer Bilderfassungsvorrichtung ("image pick-up apparatus") 30 in einem distalen Endabschnitt ("distal end portion") 11 distal eines biegbaren Abschnitts ("bending portion") des Schafts ("long insertion portion") 2 beschrieben (Absätze [0021], [0023], [0025], Fig. 1, 2).

Bei einem Rotieren einer von der Längsachse des distalen Endes des Schafts abweichenden Blickrichtung auf einen Kegelmantel um die Längsachse des distalen Endes des Schafts wird auch das von dem Endoskop erzeugte Bild relativ zu dem Endoskop rotiert. Bei einem analogen, das heißt rein optischen Endoskop, mit dessen Okular eine Kamera verbunden ist, kann dies durch eine gegenläufige Rotation der Kamera oder anders ausgedrückt durch ein Festhalten der Kamera während des Rotierens des Endoskops vermieden werden. Bei einem monokularen Videoendoskop kann das vom mitrotierenden Bildsensor erfasste Bild digital zurückrotiert werden. Bei einem Stereo-Videoendoskop rotiert jedoch mit dem Endoskop auch die Stereobasis. Auch dies kann digital kompensiert werden, indem aus dem Stereobild Tiefeninformation berechnet, also räumliche Information gewonnen und aus dieser ein Stereobild mit der gewünschten Basis synthetisiert wird. Allerdings sind der Rechenaufwand hoch und in vielen Situationen Artefakte und Bildstörungen hinzunehmen.

Eine Aufgabe der vorliegenden Erfindung besteht darin, ein verbessertes, insbesondere mechanisch robustes und miniaturisierbares Endoskop oder Exoskop mit bewegbarer Blickrichtung zu schaffen.

Diese Aufgabe wird gelöst durch den Gegenstand des unabhängigen Anspruchs 1.

Weitere Ausführungsformen sind in den abhängigen Ansprüchen definiert.

Ausführungsformen der vorliegenden Erfindung beruhen auf der Idee, eine Bilderfassungseinrichtung an einem distalen Ende eines Schaftbauteils schwenkbar, aber nicht rotierbar zu befestigen und die Schwenkposition der Bilderfassungseinrichtung durch eine Schwenksteuereinrichtung von der rotatorischen Position eines Außenschafts abhängig zu steuern.

Eine Endoskopvorrichtung umfasst ein Schaftbauteil mit einem proximalen Ende und einem distalen Ende zum Einführen in einen Hohlraum, wobei das Schaftbauteil in einem Außenschaft angeordnet ist oder zur Anordnung in einem Außenschaft vorgesehen und ausgebildet ist, eine Bilderfassungseinrichtung mit einem Objektiv zum Erzeugen eines reellen Bilds und einem Bildsensor zum Erfassen des reellen Bilds und zum Erzeugen eines Bildsignals, das das erfasste reelle Bild repräsentiert, wobei die Bilderfassungseinrichtung an dem distalen Ende des Schaftbauteils angeordnet ist, eine Schwenkgelenkeinrichtung, die die Bilderfassungseinrichtung um zwei orthogonale Achsen schwenkbar, aber hinsichtlich einer Rotation um die Längsachse des Schaftbauteils starr mit dem Schaftbauteil koppelt, und eine Schwenksteuereinrichtung zum Einstellen einer Schwenkposition der Bilderfassungseinrichtung relativ zu dem Schaftbauteil abhängig von einer rotatorischen Position des Außenschafts relativ zu der Bilderfassungseinrichtung und dem Schaftbauteil. Die Endoskopvorrichtung ist insbesondere zur Verwendung bei medizinischen Maßnahmen, vor allem bei mikroinvasiven medizinischen Maßnahmen, vorgesehen und ausgebildet. Alternativ kann die Endoskopvorrichtung für nicht-medizinische Anwendungen vorgesehen und ausgebildet sein.

Der Außenschaft kann Bestandteil der Endoskopvorrichtung sein, sodass die Endoskopvorrichtung ein vollständiges und einsetzbares Endoskop darstellt, das nur noch mit Vorrichtungen außerhalb des Operationsfelds kombiniert werden muss, insbesondere einer Lichtquelle, einer Kamerasteuerungseinheit (CCU = Camera Control Unit), einer Bildverarbeitung und einem Bildschirm. In diesem Fall kann der Außenschaft dauerhaft und nicht ohne Weiteres trennbar mit den übrigen Bauteilen des Endoskops, insbesondere mit dem Schaftbauteil mechanisch verbunden sein. Nicht ohne Weiteres trennbar bedeutet nicht ohne Verwendung von Werkzeug, das in einem Behandlungsraum oder einem Operationssaal typischerweise nicht zur Verfügung steht. Alternativ kann der Außenschaft beispielsweise zur Reinigung oder Instandsetzung abnehmbar oder zur Veränderung des Winkels zwischen Blickrichtung und Schaftachse austauschbar und dazu insbesondere ohne Werkzeug von den anderen Bauteilen der Endoskopvorrichtung trennbar sein.

Alternativ ist der Außenschaft nicht Bestandteil der Endoskopvorrichtung. In diesem Fall bildet die Endoskopvorrichtung erst zusammen mit dem Außenschaft ein vollständiges Endoskop, das - ergänzt durch Vorrichtungen außerhalb des Operationsfelds, nämlich Lichtquelle, Kamerasteuerungseinheit, Bildverarbeitungseinrichtung, Bildschirm und dergleichen - einsetzbar ist. Die Endoskopvorrichtung kann zur Verwendung mit einem Außenschaft, der lediglich einmal verwendbar ist, und/oder zur Verwendung mit einem Außenschaft, der wiederholt gereinigt, sterilisiert und wiederverwendet werden kann, vorgesehen sein. Die Endoskopvorrichtung kann zur Verwendung mit verschiedenen Außenschäften, die unterschiedliche Winkel zwischen der Blickrichtung und der Längsachse des distalen Endes des Schafts erzeugen, vorgesehen und ausgebildet sein.

Die Bilderfassungseinrichtung ist insbesondere unmittelbar proximal einer Öffnung oder in einer Öffnung am distalen Ende des Außenschafts angeordnet oder für eine Anordnung unmittelbar proximal oder in der Öffnung vorgesehen und ausgebildet. Wenn der Außenschaft ein optisch transparentes, jedoch fluiddicht oder sogar hermetisch dicht eingesetztes Fensterbauteil aufweist, ist die Bilderfassungseinrichtung insbesondere unmittelbar proximal des Fensterbauteils angeordnet.

Das Schwenken der Bilderfassungseinrichtung und damit der Blickrichtung abhängig von der rotatorischen Position des Außenschafts relativ zu der Bilderfassungseinrichtung und dem Schaftbauteil kann ein besonders intuitives Steuern der Blickrichtung ermöglichen. Ferner kann eine Rotierbarkeit des Außenschafts besonders einfach mit einer Austauschbarkeit des Außenschafts kombiniert werden, die wiederum bei der Reinigung, Instandsetzung oder Anpassung des Endoskops an eine bestimmte Anwendung vorteilhaft sein kann.

Bei einer Endoskopvorrichtung, wie sie hier beschrieben ist, umfasst die Schwenksteuereinrichtung insbesondere eine erste Gleitfläche in dem Außenschaft und eine zweite Gleitfläche, die mit der Bilderfassungseinrichtung mechanisch starr verbunden ist, zur Anlage an der ersten Gleitfläche in dem Außenschaft.

Es muss nicht jeder Teil der ersten Gleitfläche jederzeit an der zweiten Gleitfläche anliegen und nicht jeder Teil der zweiten Gleitfläche jederzeit an der ersten Gleitfläche anliegen. Als erste Gleitfläche wird jener Teil der Oberfläche des Außenschafts - insbesondere der inneren Oberfläche des Außenschafts - bezeichnet, der bei der vorgesehenen Verwendung der Endoskopvorrichtung bei irgendeiner erreichbaren Orientierung des Außenschafts relativ zu dem Schaftbauteil an der zweiten Gleitfläche anliegen kann. Als zweite Gleitfläche wird jener Teil der mit der Bilderfassungseinrichtung starr verbundenen Oberfläche bezeichnet, der bei der vorgesehenen Verwendung der Endoskopvorrichtung bei irgendeiner erreichbaren Orientierung des Außenschafts relativ zu dem Schaftbauteil an der ersten Gleitfläche anliegen kann.

Die erste Gleitfläche ist insbesondere ringförmig. Die erste Gleitfläche ist insbesondere an dem distalen Ende des Außenschafts angeordnet und nach proximal orientiert. Die zweite Gleitfläche kann ebenfalls ringförmig ausgebildet sein. Die zweite Gleitfläche kann mehrere Teilflächen umfassen, wobei eine optische Achse des Objektivs der Bilderfassungseinrichtung insbesondere zwischen den Teilflächen angeordnet ist. Die zweite Gleitfläche ist insbesondere an einem Träger oder Gehäuse der Bilderfassungseinrichtung ausgebildet. Die zweite Gleitfläche ist insbesondere im Wesentlichen nach distal orientiert.

Die Gestalt der Gleitflächen kann den Zusammenhang zwischen der rotatorischen Position des Außenschafts relativ zu der Bilderfassungseinrichtung und dem Schaftbauteil einerseits und der Orientierung der Bilderfassungseinrichtung und ihrer Blickrichtung andererseits formschlüssig definieren.

Bei einer Endoskopvorrichtung, wie sie hier beschrieben ist, ist die erste Gleitfläche insbesondere am Rand der inneren Oberfläche eines Fensterbauteils des Außenschafts angeordnet.

Bei einer Endoskopvorrichtung, wie sie hier beschrieben ist, ist insbesondere eine Flächennormale der inneren Oberfläche des Fensterbauteils relativ zu einer Längsachse des distalen Endes des Außenschafts geneigt, wobei die erste Gleitfläche eine Teilfläche der inneren Oberfläche des Fensterbauteils ist.

Insbesondere ist ein Randbereich der inneren Oberfläche des Fensterbauteils als erste Gleitfläche ausgebildet.

Wenn die innere Oberfläche des Fensterbauteils und damit insbesondere das gesamte Fensterbauteil gegenüber der Längsachse des distalen Endes des Schaftbauteils und des distalen Endes des Außenschafts gekippt ist, ist auch die erste Gleitfläche gegenüber der Längsachse gekippt. Wenn die mit der Bilderfassungseinrichtung mechanisch starr verbundene zweite Gleitfläche an der ersten Gleitfläche anliegt, weist die Bilderfassungseinrichtung und damit auch deren Blickrichtung unabhängig von der rotatorischen Position des Außenschafts eine vorbestimmte Orientierung relativ zu dem Fensterbauteil auf. Bei einer Orientierung der zweiten Gleitfläche orthogonal zu der Blickrichtung der Bilderfassungseinrichtung ist die Blickrichtung dann orthogonal zu der inneren Oberfläche des Fensterbauteils und parallel zu der Flächennormale der inneren Oberfläche des Fensterbauteils.

Bei einer Endoskopvorrichtung, wie sie hier beschrieben ist, ist die zweite Gleitfläche insbesondere an einem Rahmen oder einem Träger oder einem Gehäuse der Bilderfassungseinrichtung vorgesehen.

Beispielsweise weist die Bilderfassungseinrichtung einen becherförmigen Träger auf, an dessen im Wesentlichen nach distal orientiertem Rand die zweite Gleitfläche vorgesehen ist.

Alternativ kann die Bilderfassungseinrichtung mehrere nach distal ragende Füße oder Pfosten aufweisen, an denen jeweils eine Teilfläche der zweiten Gleitfläche vorgesehen ist.

Bei einer Endoskopvorrichtung, wie sie hier beschrieben ist, ist das Objektiv der Bilderfassungseinrichtung insbesondere zwischen zwei Teilbereichen der zweiten Gleitfläche angeordnet.

Die zwei Teilbereich der zweiten Gleitfläche können Teilbereiche einer einzigen zusammenhängenden, beispielsweise kreisringförmigen zweiten Gleitfläche sein. Alternativ können die zwei Teilbereiche voneinander beabstandet und nicht durch andere Teilbereiche der zweiten Gleitfläche verbunden sein.

Eine Endoskopvorrichtung, wie sie hier beschrieben ist, umfasst insbesondere ferner einen ersten Magneten, der mit der Bilderfassungseinrichtung mechanisch starr verbunden oder über eine Kraftübertragungseinrichtung mechanisch gekoppelt ist, und einen zweiten Magneten, der mit dem Schaftbauteil oder mit einem proximalen Bereich des Endoskops oder mit dem Außenschaft mechanisch starr verbunden ist, wobei der erste Magnet und der zweite Magnet derart angeordnet und orientiert sind, dass eine Kraft zwischen dem ersten Magneten und dem zweiten Magneten eine nach distal gerichtete Kraft auf die Bilderfassungseinrichtung ausübt.

Die von der Wechselwirkung der beiden Magneten erzeugte nach distal gerichtete Kraft auf die Bilderfassungseinrichtung kann insbesondere die zweite Gleitfläche an der Bilderfassungseinrichtung gegen die erste Gleitfläche in dem Außenschaft drücken und damit die formschlüssige Definition der Orientierung der Bilderfassungseinrichtung durch den Kontakt zwischen den Gleitflächen ermöglichen.

Wenn der erste Magnet mit der Bilderfassungseinrichtung unmittelbar mechanisch starr verbunden ist, ist der zweite Magnet insbesondere unmittelbar proximal der Bilderfassungseinrichtung an dem Schaftbauteil oder an dem Außenschaft angeordnet und stößt den ersten Magneten nach distal ab. Alternativ kann der zweite Magnet distal der Bilderfassungseinrichtung oder zumindest distal des ersten Magneten angeordnet sein und den ersten Magneten anziehen. In diesem Fall ist der erste Magnet insbesondere an einem proximalen Ende der Bilderfassungseinrichtung vorgesehen.

Alternativ ist der erste Magnet an einer Kraftübertragungseinrichtung angeordnet, beispielsweise am proximalen Ende einer Stange oder eines Rohres. Das proximale Ende der Kraftübertragungseinrichtung und der erste Magnet können am proximalen Ende des Schaftbauteils oder in einer Handhabungseinrichtung am proximalen Ende der Endoskopvorrichtung angeordnet sein. Wenn der zweite Magnet proximal des ersten Magneten angeordnet ist, sind beide Magneten so orientiert, dass der zweite Magnet den ersten Magneten abstößt. Wenn der zweite Magnet distal des ersten Magneten angeordnet ist, sind beide Magneten insbesondere so orientiert, dass der zweite Magnet den ersten Magneten anzieht. Die Kraftübertragungseinrichtung überträgt die nach distal orientierte Kraft aus der Wechselwirkung der Magneten zu der Bilderfassungseinrichtung. Das distale Ende der Kraftübertragungseinrichtung ist insbesondere gelenkig mit der Bilderfassungseinrichtung verbunden.

Eine Endoskopvorrichtung, wie sie hier beschrieben ist, umfasst insbesondere mehrere erste Magneten, die mit der Bilderfassungseinrichtung mechanisch starr verbunden oder über Kraftübertragungseinrichtungen mechanisch gekoppelt sind, wobei die ersten Magneten und der zweite Magnet derart angeordnet und orientiert sind, dass eine Kraft zwischen dem ersten Magneten und dem zweiten Magneten eine nach distal gerichtete Kraft auf die Bilderfassungseinrichtung ausübt.

Die mehreren ersten Magneten weisen insbesondere die gleiche Orientierung auf und sind symmetrisch an der Bilderfassungseinrichtung angeordnet, beispielsweise gleichmäßig über ihren Umfang verteilt.

Eine Endoskopvorrichtung, wie sie hier beschrieben ist, umfasst insbesondere mehrere zweite Magneten, die mit dem Schaftbauteil oder mit einem proximalen Bereich des Endoskops oder mit dem Außenschaft mechanisch starr verbunden sind, wobei der erste Magnet oder die ersten Magneten und die zweiten Magneten derart angeordnet und orientiert sind, dass Kräfte zwischen dem oder den ersten Magneten und dem zweiten Magneten nach distal gerichtete Kräfte auf die Bilderfassungseinrichtung ausüben.

Die zweiten Magneten sind insbesondere symmetrisch an dem Schaftbauteil oder an oder in dem Außenschaft angeordnet und gleich orientiert.

Eine Endoskopvorrichtung, wie sie hier beschrieben ist, umfasst insbesondere ferner eine Feder oder eine andere elastische Einrichtung mit einem ersten Ende, das mit der Bilderfassungseinrichtung unmittelbar mechanisch verbunden oder durch eine Kraftübertragungseinrichtung mechanisch gekoppelt ist, und einem zweiten Ende, das mit dem Schaftbauteil oder einem proximalen Bereich des Endoskops unmittelbar oder mittelbar mechanisch verbunden ist, wobei die Feder eine nach distal gerichtete Kraft auf die Bilderfassungseinrichtung ausübt.

Die durch die Feder erzeugte, nach distal gerichtete Kraft auf die Bilderfassungseinrichtung bewirkt insbesondere ein Anliegen der zweiten Gleitfläche der Bilderfassungseinrichtung an der ersten Gleitfläche des Außenschafts.

Eine Endoskopvorrichtung, wie sie hier beschrieben ist, umfasst insbesondere mehrere Federn oder andere elastische Einrichtungen mit je einem ersten Ende, das mit der Bilderfassungseinrichtung unmittelbar mechanisch verbunden oder durch eine Kraftübertragungseinrichtung mechanisch gekoppelt ist, und je einem zweiten Ende, das mit dem Schaftbauteil oder einem proximalen Bereich des Endoskops unmittelbar oder mittelbar mechanisch verbunden ist, wobei die Federn nach distal gerichtete Kräfte auf die Bilderfassungseinrichtung ausüben.

Eine Endoskopvorrichtung, wie sie hier beschrieben ist, umfasst insbesondere ferner mehrere erste Magneten, die mit der Bilderfassungseinrichtung mechanisch starr verbunden oder durch je eine Kraftübertragungseinrichtung mechanisch gekoppelt sind, und eine Magnetanordnung aus einem oder mehreren zweiten Magneten, die mit dem Außenschaft mechanisch starr verbunden ist, zum Erzeugen eines führenden Magnetfelds, das auf jeden der ersten Magneten eine Kraft in Richtung zu einer Position, die von der rotatorischen Position der Bilderfassungseinrichtung und des Schaftbauteils relativ zu dem Außenschaft abhängt, erzeugt.

Die Magnetanordnung kann eine rein magnetische und im Idealfall berührungsfreie Führung der Bilderfassungseinrichtung und bei einer Rotation des Außenschafts ein Schwenken der Bilderfassungseinrichtung und der Blickrichtung bewirken.

Bei einer Endoskopvorrichtung, wie sie hier beschrieben ist, umfasst die Kraftübertragungseinrichtung oder jede der mehreren Kraftübertragungseinrichtungen insbesondere ein stabartiges Bauteil, das in seiner Längsrichtung bewegbar in oder an dem Schaftbauteil geführt ist.

Das stabartige Bauteil ist insbesondere hinsichtlich seiner Fähigkeit, Kräfte in seiner Längsrichtung zu übertragen, stabartig. Das stabartige Bauteil ist insbesondere stabförmig mit einem kreisförmigen, rechteckigen oder beliebigen anderen Querschnitt.

Bei einer Endoskopvorrichtung, wie sie hier beschrieben ist, umfasst die Schwenkgelenkeinrichtung insbesondere ein Gelenk, das die Bilderfassungseinrichtung mit einem distalen Ende des stabartigen Bauteils verbindet.

Bei einer Endoskopvorrichtung, wie sie hier beschrieben ist, umfasst die Schwenkgelenkeinrichtung insbesondere mehrere Gelenke, wobei jedes der mehreren Gelenke die Bilderfassungseinrichtung mit einem distalen Ende von einem der stabartigen Bauteile verbindet.

Wenn die Schwenkgelenkeinrichtung genau ein Gelenk umfasst, verbindet dieses die Bilderfassungseinrichtung mit dem distalen Ende der Kraftübertragungseinrichtung insbesondere rotationssteif, unterbindet also eine Rotation der Bilderfassungseinrichtung relativ zu der Kraftübertragungseinrichtung um die Längsachse der Kraftübertragungseinrichtung.

Eine Endoskopvorrichtung, wie sie hier beschrieben ist, ist insbesondere eine Stereo-Endoskopvorrichtung, wobei die Bilderfassungseinrichtung zwei gleiche oder ähnliche Anordnungen von je einem Objektiv und einem Bildsensor umfasst, die miteinander mechanisch starr verbunden sind.

Die Bilderfassungseinrichtung kann drei oder mehr gleiche oder ähnliche Anordnungen von je einem Objektiv und einem Bildsensor umfassen. Mit mindestens zwei gleichen oder ähnlichen Anordnungen von je einem Objektiv und einem Bildsensor ermöglicht die Bilderfassungseinrichtung die Erfassung eines Stereobilds, das ein für die Betrachtung mit dem linken Auge vorgesehenes Bild und ein für die Betrachtung mit dem rechten Auge vorgesehenes Bild umfasst. Die Schwenkbarkeit der Bilderfassungseinrichtung relativ zu dem Schaftbauteil ermöglicht ein Schwenken der Blickrichtung der Bilderfassungseinrichtung ohne ein Stürzen des Horizonts und ohne ein Kippen der Stereobasis.

Ein Endoskop umfasst eine Endoskopvorrichtung, wie sie hier beschrieben ist, und einen Außenschaft.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische vergrößerte Darstellung des distalen Endes des Endoskops aus Figur 1;
- Figur 3: eine weitere schematische vergrößerte Darstellung des distalen Endes des Endoskops aus den Figuren 1 und 2;

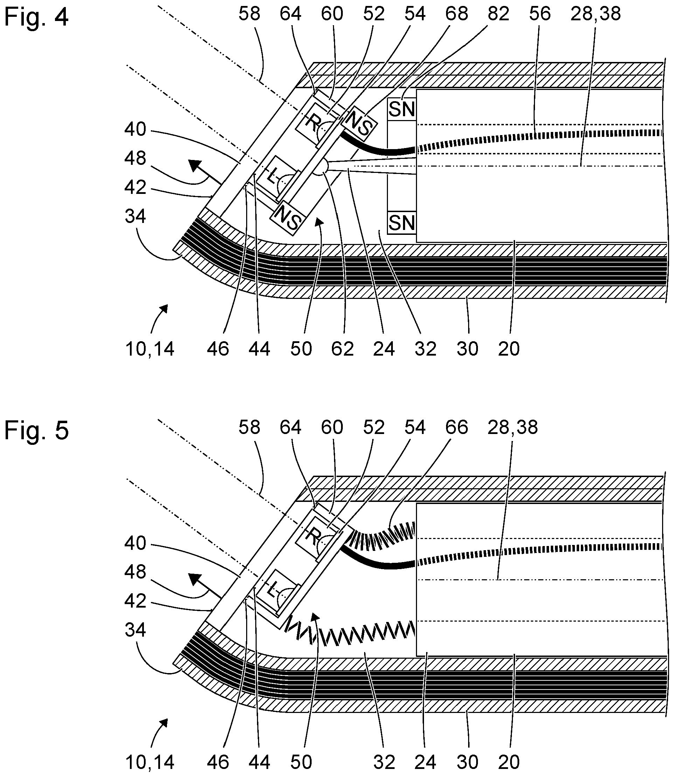

- Figur 4: eine schematische Darstellung eines distalen Endes eines weiteren Endoskops;
- Figur 5: eine schematische Darstellung eines distalen Endes eines weiteren Endoskops;

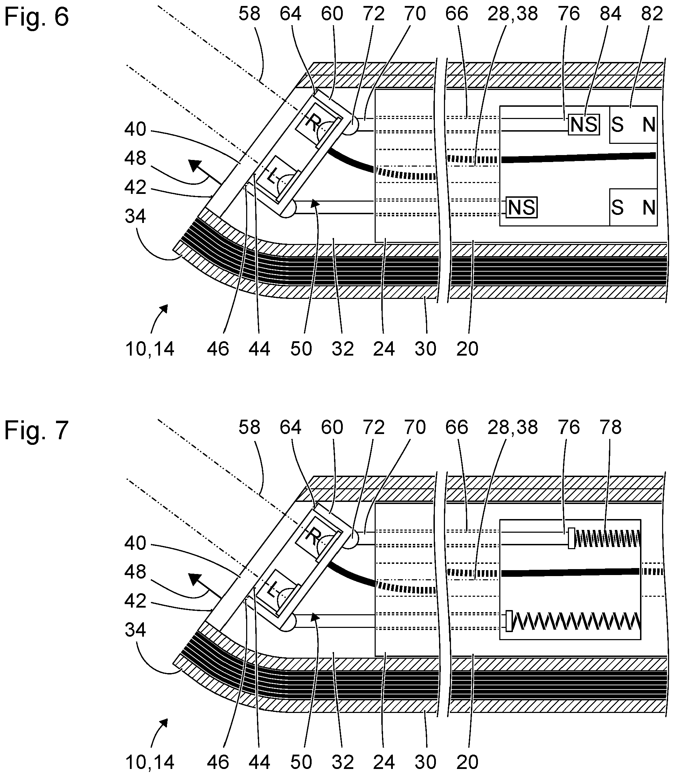

- Figur 6: eine schematische Darstellung eines distalen Endes eines weiteren Endoskops;
- Figur 7: eine schematische Darstellung eines distalen Endes eines weiteren Endoskops;

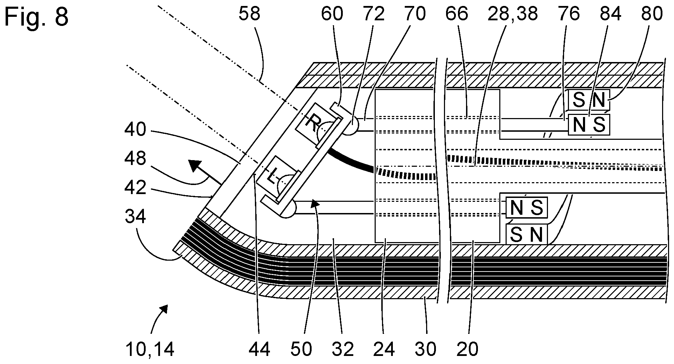

- Figur 8: eine schematische Darstellung eines distalen Endes eines weiteren Endoskops.

### Beschreibung der Ausführungsformen

Figur 1 zeigt eine schematische Darstellung eines Endoskops 10 mit einem Schaft 12, der ein distales Ende 14 zum Einführen in einen Hohlraum und ein proximales Ende 16 aufweist. An das proximale Ende 16 des Schafts 12 schließt sich ein proximaler Bereich 18 an, der als Handhabungseinrichtung ausgebildet ist. Der proximale Bereich 18 kann, wie in Figur 1 angedeutet, einen oder mehrere Steckverbinder zur Verbindung des Endoskops 10 mit einer Lichtquelle und/oder einer Leistungsquelle, einer Kamerasteuerungseinheit (CCU) und/oder einem Bildschirm aufweisen.

Der Schaft 12 des Endoskops 10 wird durch ein Schaftbauteil 20 und einen Außenschaft 30 gebildet. Beide erstrecken sich von dem distalen Ende 14 bis zu dem proximalen Ende 16 des Schafts 12. Der Außenschaft 30 umschließt rohrförmig ein Lumen 32, in dem das Schaftbauteil 20 angeordnet ist. Das proximale Ende 26 des Schaftbauteil 20 ist mit dem proximalen Bereich 18 des Endoskops mechanisch starr verbunden. Das distale Ende 34 des Außenschafts 30 ist insbesondere durch ein optisch transparentes Fensterbauteil verschlossen, sodass das Lumen 32 des Außenschafts 30 zumindest an und nahe dem distalen Ende 14 des Schafts 12 durch den Außenschaft 30 von der Umgebung des Schafts 12 getrennt ist. Das proximale Ende 36 des Außenschafts 30 umschließt das proximale Ende 26 des Schaftbauteils 20 und ist mit einem Drehrad 19 mechanisch starr verbunden. Das Drehrad 19 ermöglicht eine manuelle Rotation des Außenschafts 30 relativ zu dem Schaftbauteil 20 und dem proximalen Bereich 18 des Endoskops 10.

Das distale Ende 24 des Schaftbauteils 20 ist mit einer Bilderfassungseinrichtung 50 innerhalb des Außenschafts 30 derart mechanisch verbunden, dass die Bilderfassungseinrichtung 50 relativ zu dem Schaftbauteil 20 nicht um die Längsachse 28 des Schaftbauteils 20 rotiert, jedoch innerhalb eines vorbestimmten Raumwinkelbereichs um zwei zu der Längsachse 28 des Schaftbauteils 20 orthogonale Schwenkachsen geschwenkt werden kann. Wie nachfolgend beschrieben können durch Rotation des Außenschafts 30 um seine Längsachse 38 die Blickrichtung der Bilderfassungseinrichtung 50 auf einem Kegelmantel geschwenkt werden.

Figur 2 zeigt eine vergrößerte schematische Darstellung des distalen Endes 14 des anhand der Figur 1 dargestellten Endoskops. Die Zeichenebene der Figur 2 entspricht der Zeichenebene der Figur 1.

Der Außenschaft 30 ist in Figur 2 im Schnitt entlang einer Ebene, die die Längsachse 28 des Schaftbauteils 20 und die Längsachse 28 des Lumens 32 des Außenschafts 30 enthält, dargestellt. Es ist angedeutet, dass bei dem dargestellten Beispiel der Außenschaft 30 aus zwei ineinander angeordneten Rohren gebildet ist. Lichtleitfasern zur Übertragung von Beleuchtungslicht sind in einem Zwischenraum mit sichelförmigem Querschnitt zwischen diesen beiden Rohren angeordnet.

Das distale Ende des Außenschafts 30 ist durch ein optisch transparentes Fensterbauteil 40 hermetisch dicht verschlossen. Das Fensterbauteil 40 weist eine äußere, distale Oberfläche 42 als Lichteintrittsfläche und eine innere, proximale Oberfläche 44 als Lichtaustrittsfläche auf. Bei dem dargestellten Beispiel sind die äußere, distale Oberfläche 42 und die innere, proximale Oberfläche 44 des Fensterbauteils 40 jeweils eben und parallel zueinander.

Die Bilderfassungseinrichtung 50 weist bei dem dargestellten Beispiel zwei Objektive 52, die jeweils ein reelles Bild erzeugen, und zwei Bildsensoren 54 auf. Jeder Bildsensor 54 ist einem der beiden Objektive 52 zugeordnet. Jeder Bildsensor 54 erfasst das von dem zugeordneten Objektiv 52 erzeugte reelle Bild und erzeugt ein Bildsignal, das das erfasste reelle Bild repräsentiert. Die optischen Achsen 58 der Objektive 52 sind parallel zu der Blickrichtung der Bilderfassungseinrichtung 50.

Eine Signalleitung 56 verbindet die Bilderfassungseinrichtung 50 mit dem proximalen Bereich 18 des Endoskops 10 (vgl. Figur 1). Durch die Signalleitung 56 können Steuersignale und elektrische Leistung zu der Bilderfassungseinrichtung 50 und die von den Bildsensoren 54 erzeugten Bildsignale zu dem proximalen Bereich 18 des Endoskops 10 übertragen werden.

Die Bilderfassungseinrichtung 50 umfasst einen Träger 60, mit dem die Bildsensoren 54 und die Objektive 52 mechanisch starr verbunden sind. Der Träger 60 ist über ein Gelenk 62 mit dem distalen Ende 24 des Schaftbauteils 20 mechanisch verbunden. Das Gelenk 62 ist - beispielsweise als Kardangelenk - derart ausgebildet, dass der Träger 60 und damit die gesamte Bilderfassungseinrichtung 50 relativ zu dem Schaftbauteil 20 um zwei zu der Längsachse 28 des Schaftbauteils 20 orthogonale Schwenkachsen geschwenkt, nicht jedoch um die Längsachse 28 des Schaftbauteils 20 rotiert werden kann.

Bei dem dargestellten Beispiel ist der Träger 60 als flacher Becher ausgebildet, dessen dem Fensterbauteil 40 zugewandter Rand als ebene und kreisringförmige Gleitfläche 64 ausgebildet ist. Die Gleitfläche 64 an dem Träger 60 liegt an einer Gleitfläche 46 in einem Randbereich der inneren, proximalen Oberfläche 44 des Fensterbauteils 40 an.

Durch das Anliegen der Gleitfläche 64 des Trägers 60 der Bilderfassungseinrichtung 50 an der Gleitfläche 46 im Randbereich der inneren, proximalen Oberfläche 44 des Fensterbauteils 40 und aufgrund der symmetrischen Ausgestaltung der Bilderfassungseinrichtung 50 sind die optischen Achsen 58 der Bilderfassungseinrichtung 50 jederzeit parallel zu der Flächennormalen 48 der Oberflächen 42, 44 des Fensterbauteils 40. Eine Rotation des Außenschafts 30 relativ zu dem Schaftbauteil 20 um die Längsachse 38 des Lumens 32 des Außenschafts 30 bewirkt eine Rotation der Flächennormalen 48 der Oberflächen 42, 44 des Fensterbauteils auf einem Kegelmantel, der rotationssymmetrisch zu der Längsachse 28, 38 des Schaftbauteils 20 und des Außenschafts 30 ist. Dies bewirkt eine entsprechende Rotation der optischen Achsen 58 der Bilderfassungseinrichtung 50 auf zwei Kegelmänteln, deren Symmetrieachsen parallel zu einander und zu der Längsachse 38 des Lumens 32 des Außenschafts 30 sind.

Figur 3 zeigt eine weitere schematische Darstellung des distalen Endes 14 des Endoskops aus den Figuren 1 und 2. Die Art der Darstellung in Figur 3, insbesondere die Orientierung und Lage der Zeichen- und Schnittebenen, entspricht derjenigen der Figur 2.

Die in Figur 3 gezeigte Konfiguration unterscheidet sich von der in Figur 2 gezeigten Konfiguration dadurch, dass der Außenschaft 30 relativ zu dem Schaftbauteil 20 um 180° um die Längsachsen 28, 38 des Schaftbauteils 20 und des Außenschafts 30 rotiert ist. Entsprechend unterscheiden sich die Orientierung der Flächennormalen 48 der Oberflächen 42, 44 des Fensterbauteils 40, die Orientierung der optischen Achsen 58 und die Blickrichtung der Bilderfassungseinrichtung 50 bei der in Figur 3 gezeigten Konfiguration von der in Figur 2 gezeigten Konfiguration.

Bei der beschriebenen und in den Figuren 2 und 3 gezeigten Rotation des Außenschafts 30 und damit der optischen Achsen 58 und der Blickrichtung 58 auf Kegelmänteln wird aufgrund der rotationssteifen mechanischen Verbindung der Bilderfassungseinrichtung 50 mit dem distalen Ende 24 des Schaftbauteils 20 die Basis, also die gerade Verbindungsstrecke zwischen den Mitten der Bildsensoren 54, nicht rotiert. Die in Figur 3 oben dargestellte Einheit aus Objektiv 52 und Bildsensor 54 ist die in Figur 3 oben dargestellte Einheit aus Objektiv 52 und Bildsensor 54 zur Erfassung eines für die Betrachtung mit dem rechten Auge vorgesehenen Bilds, und die in Figur 3 oben dargestellte Einheit aus Objektiv 52 und Bildsensor 54 ist die in Figur 3 oben dargestellte Einheit aus Objektiv 52 und Bildsensor 54 zur Erfassung eines für die Betrachtung mit dem rechten Auge vorgesehenen Bilds. Um dies deutlich zu machen sind in den Figuren 2 und 3 die Objektive mit ,R' bzw.
,L' gekennzeichnet. Bei der Rotation der optischen Achsen 58 und der Blickrichtungen bleibt die Basis parallel zu ein und derselben vorbestimmten Ebene, die wiederum parallel zu der Längsachse 28, 38 des Schaftbauteils 20 und des Außenschafts 30 und zu der Zeichenebene der Figuren 2 und 3 ist.

Figur 4 zeigt eine schematische Darstellung eines distalen Endes 14 eines weiteren Endoskops, das in einigen Merkmalen, Eigenschaften und Funktionen dem anhand der Figuren 1 bis 3 dargestellten Endoskop ähnelt. Die Art der Darstellung in Figur 4 entspricht derjenigen der Figuren 2 und 3. Nachfolgend sind insbesondere Merkmale, Eigenschaften und Funktionen beschrieben, in denen das in Figur 4 gezeigte Endoskop sich von dem anhand der Figuren 1 bis 3 dargestellten Endoskop unterscheidet.

Das in Figur 4 gezeigte Endoskop weist einen Magneten 68 an dem Träger 60 der Bilderfassungseinrichtung 50 und einen Magneten 82 an dem distalen Ende 24 des Schaftbauteils 20 auf. Bei dem dargestellten Beispiel sind die Magneten 68, 82 an der Bilderfassungseinrichtung 50 und an dem distalen Ende 24 des Schaftbauteils 20 jeweils kreisringförmig ausgebildet. Die Magneten 68, 82 sind so orientiert, dass sie einander abstoßen.

Die abstoßende magnetische Wechselwirkung zwischen den Magneten 68, 82 erzeugt eine nach distal orientierte Kraft auf die Bilderfassungseinrichtung 50, die die Gleitfläche 64 des Trägers 60 gegen die Gleitfläche 46 an der inneren, proximalen Oberfläche 44 des Fensterbauteils 40 presst. Dies ermöglicht zusammen mit einem axialen Spiel der Bilderfassungseinrichtung 50 relativ zu dem distalen Ende 24 des Schaftbauteils 20, dass die Bilderfassungseinrichtung 50 jederzeit in der vorgesehenen Weise an dem Fensterbauteil 40 anliegt und somit die vorgesehene Orientierung aufweist. Bei dem dargestellten Beispiel ist dieses axiale Spiel beispielsweise in dem Gelenk 62 zwischen dem Träger 60 der Bilderfassungseinrichtung 50 und dem distalen Ende 24 des Schaftbauteils 20 vorgesehen. Wie bei dem anhand der Figuren 1 bis 3 dargestellten Endoskop ist die Verbindung zwischen der Bilderfassungseinrichtung 50 und dem distalen Ende 24 des Schaftbauteils 20 rotationssteif, sodass ein Schwenken der Blickrichtung 58 auf einem Kegelmantel nicht mit einer Rotation der Stereobasis einhergeht.

Figur 5 zeigt eine schematische Darstellung eines distalen Endes 14 eines weiteren Endoskops, das in einigen Merkmalen, Eigenschaften und Funktionen den anhand der Figuren 1 bis 4 dargestellten Endoskopen ähnelt. Die Art der Darstellung in Figur 5 entspricht der Art der Darstellung in den Figuren 2 bis 4. Nachfolgend sind insbesondere Merkmale, Eigenschaften und Funktionen des in Figur 5 gezeigten Endoskops beschrieben, in denen dieses sich von den anhand der Figuren 1 bis 4 dargestellten Endoskopen unterscheidet.

Bei dem in Figur 5 gezeigten Endoskop sind zwischen der Bilderfassungseinrichtung 50 und dem distalen Ende 24 des Schaftbauteils 20 mehrere Druckfedern 66 vorgesehen. In Figur 5 sind zwei Druckfedern 66 dargestellt. Bevorzugt sind drei oder mehr Druckfedern 66, die zu der Längsachse 28 des Schaftbauteils 20 symmetrisch angeordnet sind. Bei dem dargestellten Beispiel sind die Druckfedern 66 als Schraubenfedern ausgebildet.

Die elastischen Rückstellkräfte der Druckfedern 66 drücken die Gleitfläche 64 an dem Träger 60 der Bilderfassungseinrichtung 50 gegen die Gleitfläche 46 in dem Randbereich der inneren, proximalen Oberfläche 44 des Fensterbauteils 40.

Bei dem in Figur 5 dargestellten Beispiel ist kein zusätzliches Gelenk vorgesehen, das die Bilderfassungseinrichtung 50 rotationssteif mit dem distalen Ende 24 des Schaftbauteils 20 verbindet. Stattdessen verhindert die Biegesteifigkeit der Druckedern 66 eine Rotation der Bilderfassungseinrichtung 50 relativ zu dem distalen Ende 24 des Schaftbauteils 20. Alternativ können die Federn 66 beispielsweise in Bohrungen in dem Schaftbauteil 20 geführt sein, sodass jede Feder 66 bei maximaler Kompression fast vollständig in der zugeordneten Bohrung aufgenommen ist und sich nicht oder nur wenig seitlich verbiegen kann.

Figur 6 zeigt eine schematische Darstellung eines distalen Endes 14 eines weiteren Endoskops, das in einigen Merkmalen, Eigenschaften und Funktionen den anhand der Figuren 1 bis 5 dargestellten Endoskopen ähnelt. Die Art der Darstellung in Figur 6 entspricht der Art der Darstellung in den Figuren 2 bis 5. Nachfolgend sind insbesondere Merkmale, Eigenschaften und Funktionen des in Figur 6 gezeigten Endoskops beschrieben, in denen dieses sich von den anhand der Figuren 1 bis 5 dargestellten Endoskopen unterscheidet.

Das in Figur 6 gezeigte Endoskop weist zwei stabförmige Kraftübertragungseinrichtungen 70, die sich parallel zu der Längsachse 28, 38 des Schaftbauteils 20 und des Lumens 32 des Außenschafts 30 in dem Schaft des Endoskops erstrecken. Jede der zwei Kraftübertragungseinrichtungen 70 ist an oder, wie in Figur 6 angedeutet, in dem Schaftbauteil 20 spiel- und reibungsarm, jedoch in seiner Längsrichtung verschiebbar geführt. Das distale Ende 74 jeder Kraftübertragungseinrichtung 70 ist durch ein Gelenk 72 mit dem Träger 60 der Bilderfassungseinrichtung 50 mechanisch gelenkig verbunden. Die Kraftübertragungseinrichtungen 70 sind hinreichend biegesteif ausgebildet, um eine Rotation der Bilderfassungseinrichtung 50 relativ zu dem distalen Ende 24 des Schaftbauteils 20 um die Längsachse 28 des Schaftbauteils 20 zu unterbinden.

Am proximalen Ende 76 jeder Kraftübertragungseinrichtung 70 ist ein Magnet 84 angeordnet. Ferner weist das Endoskop zwei Magneten 82 auf, die so orientiert sind, dass sie abstoßende Kräfte auf die Magneten 84 an den proximalen Enden 76 der Kraftübertragungseinrichtungen 70 ausüben. Die Magneten 82 sind beispielsweise nahe dem proximalen Ende 26 des Schaftbauteils, nahe dem proximalen Ende 36 des Außenschafts 30 oder in dem proximalen Bereich 18 des Endoskops (vgl. Figur 1) angeordnet.

Bei dem dargestellten Beispiel sind die Magneten 82 in einem Hohlraum in dem Schaftbauteil 20 angeordnet. Abweichend von der Darstellung in Figur 6 können ein oder mehrere Magneten, die nach distal gerichtete Kräfte auf die Magneten 84 an den proximalen Enden 76 der Kraftübertragungseinrichtungen 70 angeordnet sind, mit dem Außenschaft 30 starr verbunden sein, beispielsweise als ringförmige Anordnung an der Innenseite des Außenschafts 30.

Die magnetische Wechselwirkung zwischen dem Magneten 82 und den Magneten 84 an den proximalen Enden 76 der Kraftübertragungseinrichtungen 70 erzeugt nach distal gerichtete Kräfte auf die Kraftübertragungseinrichtungen 70, die von diesen auf den Träger 60 der Bilderfassungseinrichtung 50 übertragen werden. Die Wechselwirkung zwischen den Magneten 82, 84 drückt somit die Gleitfläche 64 an dem Träger 60 der Bilderfassungseinrichtung 50 gegen die Gleitfläche 46 in dem Randbereich der inneren, proximalen Oberfläche 44 des Fensterbauteils 40. Die Magneten 82, 84 gewährleisten somit, dass die Blickrichtung der Bilderfassungseinrichtung 50 jederzeit parallel zu der Flächennormalen 48 der Oberflächen 42, 44 des Fensterbauteils 40 ist. Die biegesteife Ausgestaltung der Kraftübertragungseinrichtungen 70 gewährleistet, dass auch bei einer Rotation des Außenschafts 30 relativ zu dem Schaftbauteil 20 und trotz einer Reibungskraft zwischen den Gleitflächen 46, 64 die Bilderfassungseinrichtung 50 nicht mit dem Außenschaft 30 rotiert.

Figur 7 zeigt eine schematische Darstellung eines distalen Endes 14 eines weiteren Endoskops, das in einigen Merkmalen, Eigenschaften und Funktionen den anhand der Figuren 1 bis 5 dargestellten Endoskopen und vor allem dem anhand der Figur 6 dargestellten Endoskop ähnelt. Die Art der Darstellung in Figur 7 entspricht der Art der Darstellung in den Figuren 2 bis 6. Nachfolgend sind insbesondere Merkmale, Eigenschaften und Funktionen des in Figur 7 gezeigten Endoskops beschrieben, in denen dieses sich von dem anhand der Figur 6 dargestellten Endoskop unterscheidet.

Das in Figur 7 gezeigte Endoskop unterscheidet sich von dem anhand der Figur 6 dargestellten Endoskop insbesondere dadurch, dass zwischen den proximalen Enden 76 der Kraftübertragungseinrichtungen 70 und dem Schaftbauteil 20 Druckfedern 78 vorgesehen sind. Elastische Rückstellkräfte der Federn 78 drücken die Kraftübertragungseinrichtungen 70 nach distal und damit die Gleitfläche 64 an dem Träger 60 der Bilderfassungseinrichtung 50 gegen die Gleitfläche 46 in dem Randbereich der inneren, proximalen Oberfläche 44 des Fensterbauteils 40.

Bei dem in Figur 7 gezeigten Beispiel sind die Druckfedern 78 in einem Hohlraum in dem Schaftbauteil 20 angeordnet. Abweichend von der Darstellung in Figur 7 können die Druckfedern 78 zwischen den Kraftübertragungseinrichtungen 70 einem mit dem Schaftbauteil 20 mittelbar oder unmittelbar mechanisch starr verbundenen Bauteil angeordnet sein, beispielsweise in dem proximalen Bereich 18 des Endoskops 10 (vgl. Figur 1).

Figur 8 zeigt eine schematische Darstellung eines distalen Endes 14 eines weiteren Endoskops, das in einigen Merkmalen, Eigenschaften und Funktionen den anhand der Figuren 1 bis 5 und dargestellten Endoskopen und vor allem dem anhand der Figur 6 dargestellten Endoskop ähnelt. Die Art der Darstellung in Figur 8 entspricht der Art der Darstellung in den Figuren 2 bis 7. Nachfolgend sind insbesondere Merkmale, Eigenschaften und Funktionen des in Figur 8 gezeigten Endoskops beschrieben, in denen dieses sich von dem anhand der Figur 6 dargestellten Endoskop unterscheidet.

Das in Figur 8 gezeigte Endoskop unterscheidet sich von dem in Figur 6 gezeigten Endoskop insbesondere dadurch, dass an dem Außenschaft 30 ein ringförmig umlaufender Magnet oder mehrere eine ringförmig umlaufende Anordnung bildende Magneten 80 vorgesehen sind. Der oder die Magneten 80 liegen nicht in einer Ebene orthogonal zu der Längsachse 28, 38 des Schaftbauteils 20 und Lumens 32 des Außenschafts 30, sondern beispielsweise näherungsweise in einer dazu gekippten und zu den Oberflächen 42, 44 des Fensterbauteils 40 parallelen Ebene.

Die Magneten 80 an dem Außenschaft 30 und die Magneten 84 an den proximalen Enden 76 der Kraftübertragungseinrichtung 70 sind so orientiert, dass sie einander anziehen. Die Magneten 80 an dem Außenschaft 30 üben auf jeden Magneten 84 an dem proximalen Ende 76 einer Kraftübertragungseinrichtung 70 eine Kraft aus, deren Richtung von der Position des Magnets 84 an dem proximalen Ende 76 der Kraftübertragungseinrichtung 70 und damit von der Position der Kraftübertragungseinrichtung 70 selbst abhängt. Die Magneten 80 an dem Außenschaft 30 bewegen somit jeden Magneten 84 in eine Position, die von der rotatorischen Position des Außenschafts 30 und damit der Magneten 80 an dem Außenschaft 30 relativ zu dem Schaftbauteil 20 abhängt. Da die distalen Enden 74 der Kraftübertragungseinrichtungen 70 durch Gelenke 72 mit dem Träger 60 der Bilderfassungseinrichtung 50 mechanisch verbunden sind, steuern so die Magneten 80 an dem Außenschaft 30 die Schwenkposition des Trägers 60 und damit der gesamten Bilderfassungseinrichtung 50. Deshalb weist bei dem in Figur 8 gezeigten Endoskop der Träger 60 keine Gleitfläche auf und berührt das Fensterbauteil 40 nicht. Die Magneten 80 an dem Außenschaft 30 steuern somit die Schwenkposition der Bilderfassungseinrichtung 50 berührungslos.

Die Magneten 80 können an einem beliebigen Ort in dem Außenschaft 30 angeordnet sein, wobei die Kraftübertragungseinrichtungen 70 eine entsprechende Länge aufweisen. Die Magneten 80 können insbesondere in dem Drehrad 19 an dem proximalen Ende 36 des Außenschafts 30 angeordnet sein, wo mehr Bauraum zur Verfügung steht.

### Bezugszeichen

- **10**: **Endoskop**
- 12: Schaft des Endoskops 10
- 14: distales Ende des Schafts 12
- 16: proximales Ende des Schafts 12
- 18: proximaler Bereich des Endoskops 10
- 19: Drehrad als Bedienelement des Endoskops 10
- **20**: **Schaftbauteil** des Endoskops 10
- 22: Führungseinrichtung des Schaftbauteils 20 für eine Kraftübertragungseinrichtung ...
- 24: distales Ende des Schaftbauteils 20
- 26: proximales Ende des Schaftbauteils 20
- 28: Längsachse des Schaftbauteils 20
- **30**: **Außenschaft** des Endoskops 10
- 32: Lumen des Außenschafts 30
- 34: distales Ende des Außenschafts 30
- 36: proximales Ende des Außenschafts 30
- 38: Längsachse des Außenschafts 30
- **40**: **Fensterbauteil** an dem distalen Ende 34 des Außenschafts 30
- 42: äußere, distale Oberfläche des Fensterbauteils 40
- 44: innere, proximale Oberfläche des Fensterbauteils 40
- 46: Gleitfläche in einem Randbereich der inneren, proximalen Oberfläche 44 des Fensterbauteils 40
- 48: Flächennormale der Oberflächen 42, 44 des Fensterbauteils 40
- **50**: **Bilderfassungseinrichtung** des Endoskops 10
- 52: Objektiv der Bilderfassungeinrichtung 50, zur Erzeugung eines reellen Bilds
- 54: Bildsensor der Bilderfassungseinrichtung 50, zum Erfassen des von dem Objektiv 52 erzeugten reellen Bilds und zum Erzeugen eines Bildsignals
- 56: Signalleitung zum Übertragen von Leistung und/oder eines Steuersignals zu dem Bildsensor 54 und/oder zum Übertragen des Bildsignals von dem Bildsensor 54
- 58: optische Achse des Objektivs 52 der Bilderfassungseinrichtung 50
- 60: Träger der Bilderfassungseinrichtungen 50
- 62: Gelenk zwischen der Bilderfassungseinrichtung 50 und dem distalen Ende 24 des Schaftbauteils 20
- 64: Gleitfläche an dem Träger/Gehäuse
- 66: Druckfeder zwischen der Bilderfassungseinrichtung 50 und dem distalen Ende 24 des Schaftbauteils 20
- 68: Magnet an der Bilderfassungseinrichtung 50
- **70**: **Kraftübertragungseinrichtung** in oder an dem Schaftbauteil 20
- 72: Gelenk zwischen der Bilderfassungseinrichtung 50 und dem distalen Ende 74 der Kraftübertragungseinrichtung 70
- 74: distales Ende der Kraftübertragungseinrichtung 70
- 76: proximales Ende der Kraftübertragungseinrichtung 70
- 78: Feder an dem proximalen Ende der Kraftübertragungseinrichtung 70
- 80: Magnet an dem Außenschaft 30 des Endoskops 10
- 82: Magnet an dem Schaftbauteil 20 oder an einem proximalen Bereich 18 des Endoskops 10
- 84: Magnet an dem proximalen Ende der Kraftübertragungseinrichtung 70

## Patentansprüche

1. **Endoskopvorrichtung** (10), mit:
einem **Schaftbauteil** (20) mit einem proximalen Ende (26) und einem distalen Ende (24) zum Einführen in einen Hohlraum, wobei das Schaftbauteil (20) in einem Außenschaft (30) angeordnet ist oder zur Anordnung in einem Außenschaft (30) vorgesehen und ausgebildet ist;
einer **Bilderfassungseinrichtung** (50) mit einem Objektiv (52) zum Erzeugen eines reellen Bilds und einem Bildsensor (54) zum Erfassen des reellen Bilds und zum Erzeugen eines Bildsignals, das das erfasste reelle Bild repräsentiert, wobei die Bilderfassungseinrichtung (50) an dem distalen Ende (24) des Schaftbauteils (20) angeordnet ist;
einer **Schwenkgelenkeinrichtung** (62; 72), die die Bilderfassungseinrichtung (50) um zwei orthogonale Achsen schwenkbar, aber hinsichtlich einer Rotation um die Längsachse (28) des Schaftbauteils (20) starr mit dem Schaftbauteil (20) koppelt; **gekennzeichnet durch** eine **Schwenksteuereinrichtung** (46, 64; 80, 84) die eingerichtet ist zum Einstellen einer
Schwenkposition der Bilderfassungseinrichtung (50) relativ zu dem Schaftbauteil (20) abhängig von einer rotatorischen Position des Außenschafts (30) relativ zu der Bilderfassungseinrichtung (50) und dem Schaftbauteil (20).

2. Endoskopvorrichtung (10) nach dem vorangehenden Anspruch, bei dem die Schwenksteuereinrichtung umfasst:
eine **erste Gleitfläche** (46) in dem Außenschaft (30)
eine **zweite Gleitfläche** (64), die mit der Bilderfassungseinrichtung (50) mechanisch starr verbunden ist, zur Anlage an der **ersten Gleitfläche** (64) in dem Außenschaft (30).

3. Endoskopvorrichtung (10) nach dem vorangehenden Anspruch, bei dem
die **erste Gleitfläche** (46) **am Rand** der inneren Oberfläche (44) **eines Fensterbauteils** (40) des Außenschafts (30) angeordnet ist.

4. Endoskopvorrichtung (10) nach dem vorangehenden Anspruch, bei dem
eine **Flächennormale** (48) der inneren Oberfläche (44) des Fensterbauteils (40) relativ zu einer Längsachse (38) des distalen Endes (34) des Außenschafts (30) **geneigt** ist,
die **erste Gleitfläche** (46) eine Teilfläche der inneren Oberfläche (44) des Fensterbauteils (40) ist.

5. Endoskopvorrichtung (10) nach einem der Ansprüche 2 bis 4, bei dem
die **zweite Gleitfläche** (64) an einem Rahmen oder einem Träger (60) oder einem Gehäuse der Bilderfassungseinrichtung (50) vorgesehen ist.

6. Endoskopvorrichtung (10) nach einem der vorangehenden Ansprüche, bei dem
das **Objektiv** (52) der Bilderfassungseinrichtung (50) **zwischen** zwei Teilbereichen der zweiten Gleitfläche (64) angeordnet ist.

7. Endoskopvorrichtung (10) nach einem der vorangehenden Ansprüche, ferner mit:
einem **ersten Magneten** (68; 84), der mit der Bilderfassungseinrichtung (50) mechanisch starr verbunden oder über eine Kraftübertragungseinrichtung (70) mechanisch gekoppelt ist;
einem **zweiten Magneten** (82), der mit dem Schaftbauteil (20) oder mit einem proximalen Bereich (18) des Endoskops (10) oder mit dem Außenschaft (30) mechanisch starr verbunden ist;
wobei der erste Magnet (68; 84) und der zweite Magnet (82) derart angeordnet und orientiert sind, dass eine Kraft zwischen dem ersten Magneten (68; 84) und dem zweiten Magneten (82) eine **nach distal gerichtete Kraft** auf die Bilderfassungseinrichtung (50) ausübt.

8. Endoskopvorrichtung (10) nach dem vorangehenden Anspruch, mit:
**mehreren ersten Magneten** (68; 84), die mit der Bilderfassungseinrichtung (50) mechanisch starr verbunden oder über Kraftübertragungseinrichtungen (70) mechanisch gekoppelt sind,
wobei die ersten Magneten (68; 84) und der zweite Magnet (82) derart angeordnet und orientiert sind, dass eine Kraft zwischen den ersten Magneten (68; 84) und dem zweiten Magneten (82) eine **nach distal gerichtete Kraft** auf die Bilderfassungseinrichtung (50) ausübt.

9. Endoskopvorrichtung (10) nach einem der Ansprüche 7 und 8, mit:
**mehreren zweiten Magneten** (82), die mit dem Schaftbauteil (20) oder mit einem proximalen Bereich (18) des Endoskops (10) oder mit dem Außenschaft (30) mechanisch starr verbunden sind;
wobei der erste Magnet (68; 84) oder die ersten Magneten (68; 84) und die zweiten Magneten (82) derart angeordnet und orientiert sind, dass Kräfte zwischen dem oder den ersten Magneten (68; 84) und den zweiten Magneten (82) **nach distal gerichtete Kräfte** auf die Bilderfassungseinrichtung (50) ausüben.

10. Endoskopvorrichtung (10) nach einem der vorangehenden Ansprüche, ferner mit:
einer **Feder** (66; 78) oder einer anderen elastischen Einrichtung mit einem ersten Ende, das mit der Bilderfassungseinrichtung (50) unmittelbar mechanisch verbunden oder durch eine Kraftübertragungseinrichtung (70) mechanisch gekoppelt ist, und einem zweiten Ende, das mit dem Schaftbauteil (20) oder einem proximalen Bereich (18) des Endoskops (10) unmittelbar oder mittelbar mechanisch verbunden ist,
wobei die Feder (66; 78) eine **nach distal gerichtete Kraft** auf die Bilderfassungseinrichtung (50) ausübt.

11. Endoskopvorrichtung (10) nach dem vorangehenden Anspruch, mit:
**mehreren Federn** (66; 78) oder andere elastische Einrichtungen mit je einem ersten Ende, das mit der Bilderfassungseinrichtung (50) unmittelbar mechanisch verbunden oder durch eine Kraftübertragungseinrichtung (70) mechanisch gekoppelt ist, und je einem zweiten Ende, das mit dem Schaftbauteil (20) oder einem proximalen Bereich (18) des Endoskops (10) unmittelbar oder mittelbar mechanisch verbunden ist,
wobei die Federn (66; 78) **nach distal gerichtete Kräfte** auf die Bilderfassungseinrichtung (50) ausüben.

12. Endoskopvorrichtung (10) nach einem der Ansprüche 1 bis 6, ferner mit:
**mehreren ersten Magneten** (84), die mit der Bilderfassungseinrichtung (50) mechanisch starr verbunden oder durch je eine Kraftübertragungseinrichtung (70) mechanisch gekoppelt sind;
einer **Magnetanordnung aus einem oder mehreren zweiten Magneten** (80), die mit dem Außenschaft (30) mechanisch starr verbunden ist, zum Erzeugen eines führenden Magnetfelds, das auf jeden der ersten Magneten (84) eine Kraft in Richtung zu einer Position, die von der rotatorischen Position der Bilderfassungseinrichtung (50) und des Schaftbauteils (20) relativ zu dem Außenschaft (30) abhängt, erzeugt.

13. Endoskopvorrichtung (10) nach einem der Ansprüche 7 bis 12, bei dem
die Kraftübertragungseinrichtung oder jede der mehreren Kraftübertragungseinrichtungen ein **stabartiges Bauteil** (70), das in seiner Längsrichtung bewegbar in oder an dem Schaftbauteil (20) geführt ist, umfasst.

14. Endoskopvorrichtung (10) nach dem vorangehenden Anspruch, bei dem
die Schwenkgelenkeinrichtung mehrere **Gelenke** (72) umfasst,
jedes der mehreren Gelenke (72) die Bilderfassungseinrichtung (50) mit einem distalen Ende von einem der stabartigen Bauteile (70) verbindet.

15. Endoskopvorrichtung (10) nach einem der vorangehenden Ansprüche, wobei
die Endoskopvorrichtung (10) eine **Stereo-Endoskopvorrichtung** ist,
die Bilderfassungseinrichtung (50) **zwei** gleiche oder ähnliche Anordnungen von je einem **Objektiv** (52) und einem **Bildsensor** (54) umfasst, die miteinander mechanisch starr verbunden sind.

## Claims

1. **An endoscope apparatus** (10), comprising:
a **shaft component** (20) having a proximal end (26) and a distal end (24) for insertion into a cavity, wherein the shaft component (20) is arranged in an outer shaft (30) or is provided and designed for arrangement in an outer shaft (30);
an **image capture device** (50) having an objective lens (52) for generating a real image and an image sensor (54) for capturing the real image and for generating an image signal which represents the captured real image, wherein the image capture device (50) is arranged at the distal end (24) of the shaft component (20);
a **pivot joint device** (62; 72) which couples the image capture device (50) to the shaft component (20) in a manner pivotable about two orthogonal axes but rigid in respect of a rotation about the longitudinal axis (28) of the shaft component (20);
**characterized by** a **pivot control device** (46, 64; 80, 84) which is configured to set a pivot position of the image capture device (50) relative to the shaft component (20) depending on a rotational position of the outer shaft (30) relative to the image capture device (50) and the shaft component (20).

2. The endoscope apparatus (10) according to the preceding claim, in which the pivot control device comprises:
a **first sliding surface** (46) in the outer shaft (30)
a **second sliding surface** (64) which is mechanically rigidly connected to the image capture device (50), for abutting against the **first sliding surface** (64) in the outer shaft (30).

3. The endoscope apparatus (10) according to the preceding claim, in which the **first sliding surface** (46) is arranged **at the edge** of the inner top surface (44) **of a window component** (40) of the outer shaft (30).

4. The endoscope apparatus (10) according to the preceding claim, in which a **surface normal** (48) of the inner top surface (44) of the window component (40) is **inclined** relative to a longitudinal axis (38) of the distal end (34) of the outer shaft (30),
the **first sliding surface** (46) is a partial surface of the inner top surface (44) of the window component (40).

5. The endoscope apparatus (10) according to one of claims 2 to 4, in which the **second sliding surface** (64) is provided on a frame or a carrier (60) or a housing of the image capture device (50).

6. The endoscope apparatus (10) according to one of the preceding claims, in which
the **objective lens** (52) of the image capture device (50) is arranged **between** two partial regions of the second sliding surface (64).

7. The endoscope apparatus (10) according to one of the preceding claims, further comprising:
a **first magnet** (68; 84) which is mechanically rigidly connected to the image capture device (50) or mechanically coupled via a force transmission device (70);
a **second magnet** (82) which is mechanically rigidly connected to the shaft component (20) or to a proximal region (18) of the endoscope (10) or to the outer shaft (30);
wherein the first magnet (68; 84) and the second magnet (82) are arranged and oriented in such manner that a force between the first magnet (68; 84) and the second magnet (82) exerts a **distally directed force** on the image capture device (50).

8. The endoscope apparatus (10) according to the preceding claim, comprising:
**a plurality of first magnets** (68; 84) which are mechanically rigidly connected to the image capture device (50) or mechanically coupled via force transmission devices (70),
wherein the first magnets (68; 84) and the second magnet (82) are arranged and oriented in such manner that a force between the first magnets (68; 84) and the second magnet (82) exerts a **distally directed force** on the image capture device (50).

9. The endoscope apparatus (10) according to one of claims 7 and 8, comprising:
**a plurality of second magnets** (82) which are mechanically rigidly connected to the shaft component (20) or to a proximal region (18) of the endoscope (10) or to the outer shaft (30);
wherein the first magnet (68; 84) or the first magnets (68; 84) and the second magnets (82) are arranged and oriented in such manner that forces between the first magnet or magnets (68; 84) and the second magnets (82) exert **distally directed forces** on the image capture device (50).

10. The endoscope apparatus (10) according to one of the preceding claims, further comprising:
a **spring** (66; 78) or other elastic device having a first end which is directly mechanically connected to the image capture device (50) or mechanically coupled by a force transmission device (70), and a second end which is directly or indirectly mechanically connected to the shaft member (20) or a proximal region (18) of the endoscope (10),
wherein the spring (66; 78) exerts a **distally directed force** on the image capture device (50).

11. The endoscope apparatus (10) according to the preceding claim, comprising:
**a plurality of springs** (66; 78) or other elastic devices each having a first end which is directly mechanically connected to the image capture device (50) or mechanically coupled by a force transmission device (70), and a second end which is directly or indirectly mechanically connected to the shaft component (20) or a proximal region (18) of the endoscope (10),
wherein the springs (66; 78) exert **distally directed forces** on the image capture device (50).

12. The endoscope apparatus (10) according to one of claims 1 to 6, further comprising:
**a plurality of first magnets** (84) which are mechanically rigidly connected to the image capture device (50) or mechanically coupled by a respective force transmission device (70);
a **magnet arrangement of one or a plurality of second magnets** (80) which is mechanically rigidly connected to the outer shaft (30), for generating a guiding magnetic field which generates a force on each of the first magnets (84) in a direction towards a position which depends on the rotational position of the image capture device (50) and of the shaft component (20) relative to the outer shaft (30).

13. The endoscope apparatus (10) according to one of claims 7 to 12, in which the force transmission device or each of the plurality of force transmission devices comprises a **rod-like component** (70), which is movably guided in or on the shaft component (20) in its longitudinal direction.

14. The endoscope apparatus (10) according to the preceding claim, in which the pivot joint device comprises a plurality of **joints** (72),
each of the plurality of joints (72) connects the image capture device (50) to a distal end of one of the rod-like components (70).

15. The endoscope apparatus (10) according to one of the preceding claims, wherein
the endoscope apparatus (10) is a **stereo endoscope apparatus,**
the image capture device (50) comprises **two** identical or similar arrangements of respectively one **objective lens** (52) and one **image sensor** (54) which are mechanically rigidly connected to one another.

## Revendications

1. **Dispositif endoscopique** (10), comprenant :
un **composant d'arbre** (20) ayant une extrémité proximale (26) et une extrémité distale (24) pour insertion dans une cavité, dans lequel le composant d'arbre (20) est agencé dans un arbre externe (30) ou est prévu et conçu pour être agencé dans un arbre externe (30) ;
un **appareil de capture d'image** (50) ayant une lentille (52) pour générer une image réelle et un capteur d'image (54) pour capturer l'image réelle et pour générer un signal d'image, qui représente l'image réelle capturée, dans lequel l'appareil de capture d'image (50) est agencé au niveau de l'extrémité distale (24) du composant d'arbre (20) ;
un **appareil à joint pivotant** (62 ; 72) qui accouple de manière pivotante l'appareil de capture d'image (50) au composant d'arbre (20) autour de deux axes orthogonaux mais rigidement par rapport à une rotation autour de l'axe longitudinal (28) du composant d'arbre (20) ;
**caractérisé par** un **appareil de commande pivotant** (46, 64 ; 80, 84) qui est conçu pour régler une position de pivotement de l'appareil de capture d'image (50) par rapport au composant d'arbre (20) en fonction d'une position de rotation de l'arbre externe (30) par rapport à l'appareil de capture d'image (50) et au composant d'arbre (20).

2. Dispositif endoscopique (10) selon la revendication précédente, dans lequel le dispositif de commande pivotant comprend :
une **première surface de glissement** (46) dans l'arbre externe (30)
une **deuxième surface de glissement** (64), qui est reliée mécaniquement rigidement à l'appareil de capture d'image (50), pour reposer sur la **première surface de glissement** (64) dans l'arbre externe (30).

3. Dispositif endoscopique (10) selon la revendication précédente, dans lequel
la **première surface de glissement** (46) est agencée **au bord** de la surface interne (44) **d'un composant de fenêtre** (40) de l'arbre externe (30).

4. Dispositif endoscopique (10) selon la revendication précédente, dans lequel
une **normale à la surface** (48) de la surface interne (44) du composant de fenêtre (40) est **inclinée** par rapport à un axe longitudinal (38) de l'extrémité distale (34) de l'arbre externe (30),
la **première surface de glissement** (46) est une surface partielle de la surface interne (44) du composant de fenêtre (40).

5. Dispositif endoscopique (10) selon l'une quelconque des revendications 2 à 4, dans lequel
la **deuxième surface de glissement** (64) est prévue sur un cadre ou un support (60) ou un boîtier de l'appareil de capture d'image (50).

6. Dispositif endoscopique (10) selon l'une quelconque des revendications précédentes, dans lequel
le **lentille** (52) de l'appareil de capture d'image (50) est agencée **entre** deux zones partielles de la deuxième surface de glissement (64).

7. Dispositif endoscopique (10) selon l'une quelconque des revendications précédentes, comprenant en outre :
un **premier aimant** (68 ; 84) qui est relié mécaniquement rigidement à l'appareil de capture d'image (50) ou couplé mécaniquement par l'intermédiaire d'un appareil de transmission de force (70) ;
un **deuxième aimant** (82) qui est relié au composant d'arbre (20) ou à une zone proximale (18) de l'endoscope (10) ou à l'arbre externe (30) mécaniquement de manière rigide ;
dans lequel le premier aimant (68 ; 84) et le deuxième aimant (82) sont agencés et orientés de sorte qu'une force entre le premier aimant (68 ; 84) et le deuxième aimant (82) soit une **force orientée distalement** sur l'appareil de capture d'image (50).

8. Dispositif endoscopique (10) selon la revendication précédente, comprenant :
**plusieurs premiers aimants** (68 ; 84), qui sont reliés mécaniquement rigidement à l'appareil de capture d'image (50) ou couplés mécaniquement par l'intermédiaire d'appareils de transmission de force (70),
dans lequel les premiers aimants (68 ; 84) et le deuxième aimant (82) sont agencés et orientés de sorte qu'une force entre les premiers aimants (68 ; 84) et le deuxième aimant (82) soit une **force orientée distalement** sur l'appareil de capture d'image (50).

9. Dispositif endoscopique (10) selon l'une quelconque des revendications 7 et 8, comprenant :
**plusieurs deuxièmes aimants** (82) qui sont reliés mécaniquement rigidement au composant d'arbre (20) ou à une région proximale (18) de l'endoscope (10) ou à l'arbre externe (30) ;
dans lequel le premier aimant (68 ; 84) ou les premiers aimants (68 ; 84) et les deuxièmes aimants (82) sont agencés et orientés de sorte que les forces entre le ou les premiers aimants (68 ; 84) et les deuxièmes aimants (82) soient des **forces orientées distalement** sur l'appareil de capture d'image (50).

10. Dispositif endoscopique (10) selon l'une quelconque des revendications précédentes, comprenant en outre :
un **ressort** (66 ; 78) ou un autre appareil élastique ayant une première extrémité, qui est reliée mécaniquement directement à l'appareil de capture d'image (50) ou couplée mécaniquement par un appareil de transmission de force (70) et une seconde extrémité, qui est reliée mécaniquement directement ou indirectement au composant d'arbre (20) ou à une zone proximale (18) de l'endoscope (10),
dans lequel le ressort (66 ; 78) exerce un **force orientée distalement** sur l'appareil de capture d'image (50).

11. Dispositif endoscopique (10) selon la revendication précédente, comprenant :
**plusieurs ressorts** (66 ; 78) ou autres appareils élastiques ayant chacun une première extrémité, qui est reliée mécaniquement directement à l'appareil de capture d'image (50) ou couplée mécaniquement par un appareil de transmission de force (70) et chacun une seconde extrémité qui est reliée mécaniquement directement ou indirectement au composant d'arbre (20) ou à une région proximale (18) de l'endoscope (10),
dans lequel les ressorts (66 ; 78) exercent des **forces orientées distalement** sur l'appareil de capture d'image (50).

12. Dispositif endoscopique (10) selon l'une quelconque des revendications 1 à 6, comprenant en outre :
**plusieurs premiers aimants** (84) qui sont reliés mécaniquement rigidement à l'appareil de capture d'image (50) ou couplés mécaniquement chacun par un appareil de transmission de force (70) ;
un **agencement d'aimants composé d'un ou de plusieurs deuxièmes aimants** (80), qui est relié mécaniquement rigidement à l'arbre externe (30), pour générer un champ magnétique de guidage, qui génère une force sur chacun des premiers aimants (84) dans une direction vers une position qui dépend de la position de rotation de l'appareil de capture d'image (50) et du composant d'arbre (20) par rapport à l'arbre externe (30).

13. Dispositif endoscopique (10) selon l'une quelconque des revendications 7 à 12, dans lequel
l'appareil de transmission de force ou chacun de la pluralité d'appareils de transmission de force comprend un **composant en forme de tige** (70), qui est guidé de manière mobile dans ou sur le composant d'arbre (20) dans sa direction longitudinale.

14. Dispositif endoscopique (10) selon la revendication précédente, dans lequel
l'appareil à joint pivotant comprend plusieurs **articulations** (72),
chacune de la pluralité d'articulations (72) relie l'appareil de capture d'image (50) à une extrémité distale de l'un des composants en forme de tige (70).

15. Dispositif endoscopique (10) selon l'une quelconque des revendications précédentes, dans lequel
le dispositif endoscopique (10) est un **dispositif stéréo-endoscopique,**
l'appareil de capture d'image (50) comprend **deux** arrangements identiques ou similaires de chacun une **lentille** (52) et un **capteur d'image** (54), qui sont reliés mécaniquement rigidement l'un à l'autre.
